# EUROPEAN PATENT APPLICATION

(11) **EP 4 722 872 A2**
(43) Date of publication of application: **08.04.2026**
(21) Application number: 26159098.8
(22) Date of filing: 05.06.2023
(51) Int. Cl.: G06F 3/03

(54) **AN EYE TRACKING SYSTEM**

(30) Priority: 22.06.2022 SE 2250765
(62) Divisional of application: 23177171.8
(71) Applicant: Tobii AB, 182 17 Danderyd (SE)
(72) Inventor: Rana, Pravin Kumar, 18217 Danderyd (SE); Wang, Yimu, 18217 Danderyd (SE); Johansson Tornéus, Daniel, 18217 Danderyd (SE); Remon Salazar, Gilfredo, 18217 Danderyd (SE)

(57) **Abstract**

A method for calibrating an illuminance level of a plurality of light sources (441) in an eye tracking system (300), the method comprising the steps of: setting a first current level to a one or more of the plurality of light sources (310-319), illuminating at least a first surface (438) by the one or more of the plurality of light sources (310-319), determining illumination contribution of the one or more of the plurality of light sources (310-319) on the illuminance level of the eye tracking system (300), configuring the illuminance level of the eye tracking system (300) by setting an adjusted current level on the one or more of the plurality of light sources (310-319).

## Description

### Field

The present disclosure generally relates to the field of eye tracking. In particular, the present disclosure relates to controllers, algorithms, eye tracking systems and methods for improving the evenness with which a plurality of light sources illuminate a user's eye when the eye tracking system is in use.

### Background

In eye tracking applications, digital images are retrieved of the eyes of a user and the digital images are analysed in order to estimate gaze direction of the user. The estimation of the gaze direction may be based on computer-based image analysis of features of the imaged eye. One known example method of eye tracking includes the use of infrared light and an image sensor. The infrared light is directed towards eye(s) of a user and the reflection of the light is captured by an image sensor.

Many eye tracking systems estimate gaze direction based on identification of a pupil position together with glints or corneal reflections in the digital images. However, gaze estimation techniques can suffer from errors due to assumptions about the shape and / or position of the features of the eye. Therefore, improving the accuracy of such feature determination can be important for eye tracking systems and methods.

Portable or wearable eye tracking devices have been previously described. One such eye tracking system is described in U.S. Pat. No. 9,041,787 (which is hereby incorporated by reference in its entirety). A wearable eye tracking device is described using illuminators and image sensors for determining gaze direction.

### Summary

According to a first aspect of the present disclosure there is provided an eye tracking system comprising:
a plurality of light sources that are arranged to illuminate a user's eye when the eye tracking system is in use; and
a controller configured to:
   receive a first-image of a surface, acquired while the surface is illuminated by a first set of the plurality of light sources;
   receive a second-image of the surface, acquired while the surface is illuminated by a second set of the plurality of light sources, wherein the second set of light sources is different to the first set of light sources;
   process the first-image and the second-image to determine an illumination contribution of one or more of the light sources; and
   determine light-source-control-signalling for one or more of the light sources based on the determined illumination contribution of the one or more of the light sources, wherein the light-source-control-signalling is for setting the intensity of light provided by the respective light sources when the eye tracking system is in use.

Advantageously, setting the light-source-control-signalling in this way can enable the light sources to more evenly illuminate the user's eye when the eye tracking system is in use such that improved eye tracking can be achieved.

The first set of the plurality of light sources may comprise all of the light sources. The second set of the plurality of light sources may comprise a subset of the light sources. The controller may be configured to:
process the first-image and the second-image to determine an illumination contribution of the light sources that are not included in the second set of the plurality of light sources; and
determine light-source-control-signalling for the light sources that are not included in the second set of the plurality of light sources based on the determined illumination contribution of the light sources that are not included in the second set of the plurality of light sources.

The controller may be configured to:
receive a plurality of second-images, acquired while the surface is illuminated by seconds sets of the plurality of light sources that represent different subsets of the plurality of light sources;
process the first-image and the plurality of second-images to determine a plurality of illumination contributions of a plurality of the light sources; and
determine light-source-control-signalling for the light sources based on the determined plurality of illumination contributions of the plurality of the light sources.

The controller may be configured to:
determine a first-illumination-level that represents an illumination level of the first-image;
determine a second-illumination-level that represents an illumination level of the second-image; and
process the first-illumination-level and the second-illumination-level to determine the illumination contribution the one or more of the light sources.

The controller may be configured to:
determine the first-illumination-level by calculating the average intensity of pixels in the first-image; and
determine the second-illumination-level by calculating the average intensity of pixels in the second-image.

The controller may be configured to:
determine the illumination contribution of one or more of the light sources based on: a ratio of the first-illumination-level to the second-illumination-level; or the difference between the first-illumination-level and the second-illumination-level.

The controller may be further configured to:
receive a third-image of the surface, acquired while the surface is not illuminated by any of the plurality of light sources; and
process the first-image, the second-image and the third-image to determine the illumination contribution of one or more of the light sources.

The controller may be configured to:
determine a first-illumination-level that represents an illumination level of the first-image;
determine a second-illumination-level that represents an illumination level of the second-image;
determine a third-illumination-level that represents an illumination level of the third-image;
subtract the third-illumination-level from the first-illumination-level to determine an effective-first-illumination-level;
subtract the third-illumination-level from the second-illumination-level to determine an effective-second-illumination-level; and
process the effective-first-illumination-level and the effective-second-illumination-level to determine the illumination contribution of the one or more of the light sources.

The plurality of light sources may comprise a plurality of LEDS.

The light-source-control-signalling may be for setting a current level that is to be applied to the one or more light sources when the eye tracking system is in use.

The controller may be configured to:
determine the light-source-control-signalling for the one or more of the light sources by:
comparing: (i) the determined illumination contribution of one or more of the light sources with (ii) one or more threshold values, to determine an illumination-error-value; and
determining the light-source-control-signalling based on the illumination-error-value.

The one or more threshold values may not be the same for each of the plurality of light sources.

The controller may be configured to:
set the light-source-control-signalling based on the illumination-error-value by:
using a look-up table; or
applying an algorithm to the illumination-error-value.

The controller may be configured to:
drive the first set of light sources according to the determined light-source-control-signalling and receive an updated-first-image of the surface, acquired while the surface is illuminated by the first set of the plurality of light sources;
drive the second set of light sources according to the determined light-source-control-signalling and receive an updated-second-image of the surface, acquired while the surface is illuminated by the second set of the plurality of light sources;
process the updated-first-image and the updated-second-image to determine an updated illumination contribution of one or more of the light sources; and
determine updated-light-source-control-signalling for one or more of the light sources based on the determined updated illumination contribution of the one or more of the light sources.

The controller may be configured to repeatedly drive the plurality of light sources according to the updated-light-source-control-signalling to iteratively determine updated-light-source-control-signalling until an end condition is satisfied.

According to a further aspect of the present disclosure, there is provided a method of operating an eye tracking system. The eye tracking system comprising a plurality of light sources that are arranged to illuminate a user's eye when the eye tracking system is in use. The method comprising:
receiving a first-image of a surface, acquired while the surface is illuminated by a first set of the plurality of light sources;
receiving a second-image of the surface, acquired while the surface is illuminated by a second set of the plurality of light sources, wherein the second set of light sources is different to the first set of light sources;
processing the first-image and the second-image to determine an illumination contribution of one or more of the light sources; and
determining light-source-control-signalling for one or more of the light sources based on the determined illumination contribution of the one or more of the light sources, wherein the light-source-control-signalling is for setting the intensity of light provided by the respective light sources when the eye tracking system is in use.

According to a further aspect of the present disclosure, there is provided a test rig for an eye tracking system. The eye tracking system comprises a plurality of light sources that are arranged to illuminate a user's eye when the eye tracking system is in use. The test rig comprises:
a first surface of known reflectivity for simulating the reflectivity of the user's eye;
a second surface of known reflectivity, which is less reflective than the reflectivity of the first surface, for simulating the reflectivity of regions of the user's face around their eye; and
a mount for the eye tracking system such that, when the eye tracking system is located in the mount, the plurality of light sources illuminate at least the first surface.

The first surface may be generally circular. The second surface may surround the generally circular first surface.

The test rig may comprise two first surfaces, one for each eye.

The first surface may be coplanar with the second surface.

There may be provided a computer program, which when run on a computer, causes the computer to configure any apparatus, including a controller, system, or device disclosed herein or perform any method or algorithm disclosed herein. The computer program may be a software implementation, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

The computer program may be provided on a computer readable medium, which may be a physical computer readable medium such as a disc or a memory device, or may be embodied as a transient signal. Such a transient signal may be a network download, including an internet download. There may be provided one or more non-transitory computer-readable storage media storing computer-executable instructions that, when executed by a computing system, causes the computing system to perform any method disclosed herein.

### Brief Description of the Drawings

One or more embodiments will now be described by way of example only with reference to the accompanying drawings in which:
Figure 1 shows a simplified view of an eye tracking system;
Figure 2 shows a simplified example of an image of a pair of eyes, captured by an eye tracking system such as the system of Figure 1;
Figure 3 shows an example embodiment of an eye tracking system according to the present disclosure;
Figures 4A and 4B show an example embodiment of a test rig for an eye tracking system, such as the eye tracking system of Figure 3;
Figure 4C shows an example of an image that can be acquired by the camera of the eye tracking system of Figure 3 when it is located in the test rig of Figure 4A; and
Figure 5 illustrates an example embodiment of a method for calibrating an eye tracking system according to the present disclosure.

### Detailed Description

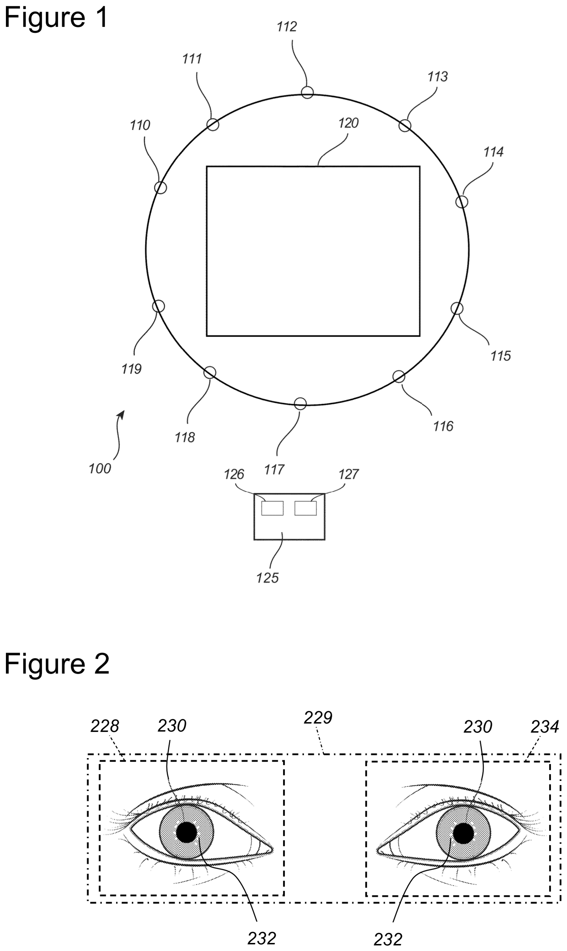

Figure 1 shows a simplified view of an eye tracking system 100 (which may also be referred to as a gaze tracking system) in a head-mounted device in the form of a virtual or augmented reality (VR or AR) device or VR or AR glasses or anything related, such as extended reality (XR) or mixed reality (MR) headsets. The system 100 comprises an image sensor 120 (e.g., a camera) for capturing images of the eyes of the user. The system may optionally include one or more illuminators 110-119 (also referred to herein as light sources) for illuminating the eyes of a user, which may for example be light emitting diodes (LEDs) emitting light in the infrared frequency band, or in the near infrared frequency band and which may be physically arranged in a variety of configurations. The image sensor 120 may for example be an image sensor of any type, such as a complementary metal oxide semiconductor (CMOS) image sensor or a charged coupled device (CCD) image sensor. The image sensor may consist of an integrated circuit containing an array of pixel sensors, each pixel containing a photodetector and an active amplifier. The image sensor may be capable of converting light into digital signals. In one or more examples, it could be an infrared image sensor or IR image sensor, an RGB sensor, an RGBW sensor or an RGB or RGBW sensor with IR filter.

The eye tracking system 100 may comprise circuitry or one or more controllers 125, for example including a receiver 126 and processing circuitry 127, for receiving and processing the images captured by the image sensor 120. The circuitry 125 may for example be connected to the image sensor 120 and the optional one or more illuminators 110-119 via a wired or a wireless connection and be co-located with the image sensor 120 and the one or more illuminators 110-119 or located at a distance, e.g., in a different device. In another example, the circuitry 125 may be provided in one or more stacked layers below the light sensitive surface of the light sensor 120.

The eye tracking system 100 may include a display (not shown) for presenting information and/or visual stimuli to the user. The display may comprise a VR display which presents imagery and substantially blocks the user's view of the real-world or an AR display which presents imagery that is to be perceived as overlaid over the user's view of the real-world.

The location of the image sensor 120 for one eye in such a system 100 is generally away from the line of sight for the user in order not to obscure the display for that eye. This configuration may be, for example, enabled by means of so-called hot mirrors which reflect a portion of the light and allows the rest of the light to pass, e.g., infrared light is reflected, and visible light is allowed to pass.

While in the above example the images of the user's eye are captured by a head-mounted image sensor 120, in other examples the images may be captured by an image sensor that is not head-mounted. Such a non-head-mounted system may be referred to as a remote system.

In an eye tracking system, a gaze signal can be computed for each eye of the user (left and right). The quality of these gaze signals can be reduced by disturbances in the input images (such as image noise) and by incorrect algorithm behaviour (such as incorrect predictions). A goal of the eye tracking system is to deliver a gaze signal that is as good as possible, both in terms of accuracy (bias error) and precision (variance error). For many applications it can be sufficient to deliver only one gaze signal per time instance, rather than both the gaze of the left and right eyes individually. Further, the combined gaze signal can be provided in combination with the left and right signals. Such a gaze signal can be referred to as a combined gaze signal.

Figure 2 shows a simplified example of an image 229 of a pair of eyes, captured by an eye tracking system such as the system of Figure 1. The image 229 can be considered as including a right-eye-image 228, of a person's right eye, and a left-eye-image 234, of the person's left eye. In this example the right-eye-image 228 and the left-eye-image 234 are both parts of a larger image of both of the person's eyes. In other examples, separate image sensors may be used to acquire the right-eye-image 228 and the left-eye-image 234. In other examples, multiple image sensors may be used to acquire images capturing both eyes.

The system may employ image processing (such as digital image processing) for extracting features in the image. The system may for example identify a position of the pupil 230 in the one or more images captured by the image sensor. The system may determine the position of the pupil 230 using a pupil detection process. The system may also identify corneal reflections 232 located in close proximity to the pupil 230. The system may estimate a corneal centre based on the corneal reflections 232. For example, the system may match each of the individual corneal reflections 232 for each eye with a corresponding illuminator and determine the corneal centre of each eye based on the matching. To a first approximation, the eye tracking system may determine an optical axis of the eye of the user as the vector passing through a centre of the pupil 230 and the corneal centre. The direction of gaze corresponds to the axis from the fovea of the eye through the centre of the pupil (visual axis). The angle between the optical axis and the gaze direction is the *foveal offset,* which typically varies from user to user and is in the range of a few degrees. The eye tracking system may perform a calibration procedure, instructing the user to gaze in a series of predetermined directions (e.g., via instructions on a screen), to determine the fovea offset. The determination of the optical axis described above is known to those skilled in the art and often referred to as pupil centre corneal reflection (PCCR). PCCR is not discussed in further detail here.

However, due to variations in production, the illuminators / light sources (which will be described in relation to the specific example of LEDs below) may not be equally bright, such that some LEDs may shine brighter than others. This can cause certain parts of the image to receive a higher illuminance and consequently result in brighter or dimmer regions of the image. The brightness of the LEDs can vary due to their own efficiency, mechanical placement, LED driver variation, hot mirror reflectance, and other causes. To achieve good eye tracking performance it is desired that eye tracking images are evenly illuminated, preferably with a desired absolute level.

To mitigate against problems that can arise due to an uneven spread of illumination, examples of the present disclosure relate to a method of calibrating the illuminance level of the LEDs (or other light sources) in the eye tracking system. Such calibration can be performed in a controlled environment, for instance using a test rig, or can be performed on images that are acquired while the eye tracking system is in use (i.e., during run-time).

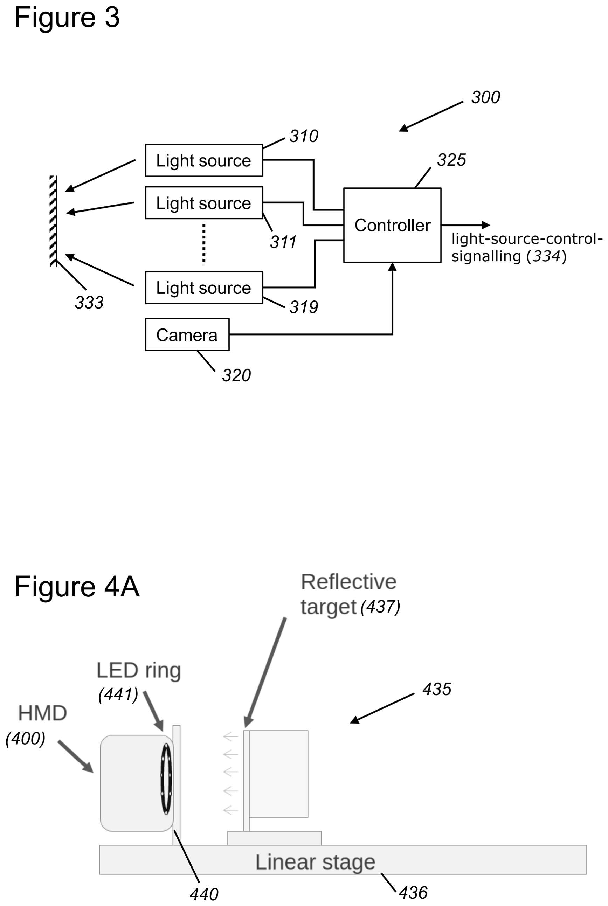

Figure 3 shows an example embodiment of an eye tracking system 300 according to the present disclosure. The eye tracking system 300 includes a plurality of light sources that are arranged to illuminate a user's eye when the eye tracking system is in use. The plurality of light sources in this example is ten LEDs 310-319 (although only three are shown in Figure 3 to assist with the clarity of the illustration). The ten LEDs 310-319 can be arranged in a circular arrangement around a user's single eye, as shown in Figure 1 with references 110-119. As discussed above, it will be appreciated that the eye tracking system 300 can include another plurality of light sources / LEDs for separately illuminating the user's other eye. The eye tracking system 300 also includes a camera 320 (as an example of an image sensor) for acquiring images of the user's eye when it is illuminated by the LEDs 310-319.

In Figure 3, the LEDs 310-319 are schematically shown as illuminating a surface 333. As will be appreciated from the description that follows, depending upon how and when the eye tracking system is calibrated, the surface 333 may be: a surface of a test rig; a surface of the user's eye that is to be tracked; or the surface of a larger portion of the user's face that includes at least one of their eyes (which is most likely in a remote system where the camera 320 and / or LEDs 310-319 are not necessarily in a fixed relationship with respect to the user's face as they can be in a head mounted device).

In the description that follows, we will only describe the functionality of eye tracking systems as they relate to determining the gaze of a single eye of a user. However, it will be appreciated that the described functionality can easily be repeated for the user's other eye.

The eye tracking system 300 of Figure 3 also includes a controller 325. The controller 325 receives a first-image of the surface 333, acquired while the surface is illuminated by a first set of the plurality of LEDs 310-319. The controller 325 also receives a second-image of the surface 333, acquired at a different point in time while the surface 333 is illuminated by a second set of the plurality of LEDs 310-319. The second set of LEDs is different to the first set of LEDs. In this way, at least one of the LEDs 310-319 is on while one of the first-image and / or the second-image is acquired, and is off while the second-image is acquired. The controller 320 can provide signalling to each of the plurality of LEDs 310-319 to either turn them on or off such that the images can be acquired with the desired set of LEDs illuminated.

The controller 325 can then process the first-image and the second-image to determine an illumination contribution of one or more of the LEDs. For instance, as will be discussed below, the brightness of the second-image can be subtracted from the brightness of the first-image in order to determine the illumination contribution of LEDs that are in the first set (and therefore are on while the first-image is acquired) but are not in the second set (and therefore are off while the second-image is acquired).

The controller 325 can then determine light-source-control-signalling for one or more of the LEDs 310-319 based on the determined illumination contribution of the one or more of the LEDs. The light-source-control-signalling is for setting the intensity of light provided by the respective LEDs when the eye tracking system is subsequently in use. In this way, the controller 325 can set the light-source-control-signalling such that the LEDs 310-319 will more evenly illuminate the user's eye and improved eye tracking can be achieved.

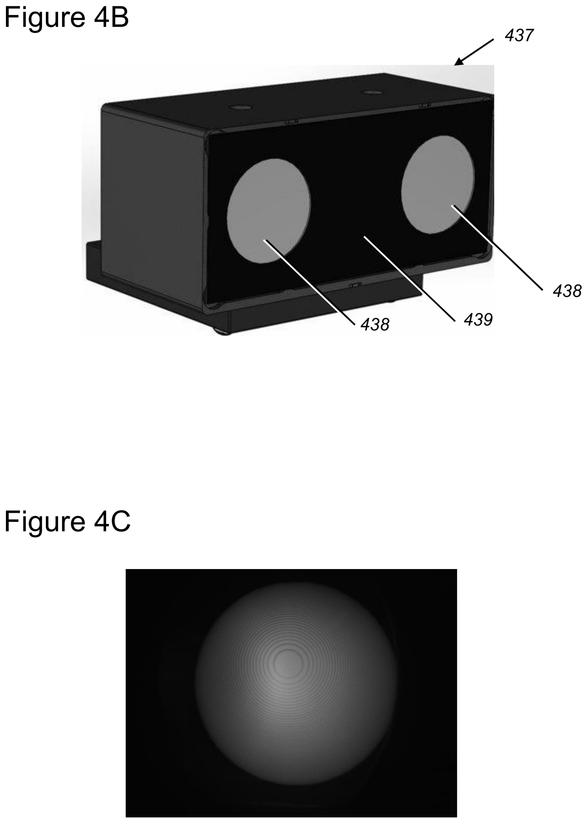

Figures 4A and 4B show an example embodiment of a test rig 435 for an eye tracking system 400, such as the eye tracking system of Figure 3. In this example the eye tracking system 400 is a head mounted device (HMD) which includes at least one ring of LEDs 441 (which can be implemented as a carrier or substrate having a substantially ring-shape on which the LEDs 441 are disposed or mounted) and a camera (not shown). As discussed above, the ring of LEDs 441 is arranged to illuminate a user's eye when the eye tracking system 400 is in use. It will be appreciated that the LEDs in the ring do not need to be positioned in a perfect circle, and also that the LEDs do not need to be equally spaced around the ring. Therefore, the LEDs can be considered as being positioned in substantially a ring formation.

The test rig 435 includes a mount 440 (that is schematically illustrated in Figure 4A) for the eye tracking system 400 such that, when the eye tracking system 400 is located in the mount 440, it is located in a desired location and orientation with respect to a reflective target 437. The reflective target 437 will be used to mimic user's eyes to simulate how light would be reflected by the eye, such that the illumination contribution of the LEDs 441 can be established and then the light-source-control-signalling can be determined for one or more of the LEDs such that the intensity of light provided by the respective LEDs when the eye tracking system is subsequently in use 400 can be adjusted, if required.

The reflective target 437 includes a first surface 438 (in this example two first surfaces 438) of known reflectivity for simulating the reflectivity of the user's eye. The reflective target 437 also includes a second surface 439 of known reflectivity, which is less reflective than the reflectivity of the first surface 438, for simulating the reflectivity of regions of the user's face around their eye(s). The first surfaces 438 are generally circular, in order to mimic the general shape of a user's eye. The second surface 439 surrounds the generally circular first surfaces 438.

When the eye tracking system 400 is located in the mount 440, the ring of LEDs 441 illuminates at least the first surface 438. In a practical sense, due to the nature of the LEDs 441, the second surface will at least partially be illuminated by the LEDs 441 too. This is equivalent to the fact that when the eye tracking system 400 is in use, the LEDs 441 will inevitably illuminate regions of the user's face in addition to their eyes. In this example, the first surfaces 438 are coplanar with the second surface 439.

As shown in Figure 4A, in this example the reflective target 457 is movable relative to the mount 440 to adjust the distance between the two components (and potentially also their relative orientation) to simulate different use cases for the eye tracking system 400 or different types of eye tracking systems 400. In the example of Figure 4A, the reflective target 437 is movable towards or away from the mount 440 along a linear stage 436.

Figure 4C shows an example of an image that can be acquired by the camera of the eye tracking system 400 when it is located in the test rig 435 of Figure 4A. The image illustrates only one the first surfaces 438 of the reflective target 437, on the basis that the camera in this eye tracking system is for acquiring an image of a single eye when it is in use. A separate camera can be provided for the other eye / first surface 438.

Returning to Figure 3, we will now describe an illustrative example of how the light-source-control-signalling can be determined by the controller 325.

In this example, the first set of the plurality of LEDs comprises all of the LEDs 310-319. That is, the first-image of the surface 333 is acquired while the surface 333 is illuminated by all of the LEDs 310-319. The second set of the plurality of LEDS comprises a subset (i.e., not all) of the LEDs 310-319. The controller 325 can then process the first-image and the second-image to determine an illumination contribution of the LEDs 310-319 that are not included in the second set of the plurality of LEDs 310-319, and determine the light-source-control-signalling for at least the LEDs 310-319 that are not included in the second set. That is, the controller can set the light-source-control-signalling to adjust the illumination of at least the LEDs 310-319 that have been isolated by including them in the first set but not the second set.

In this implementation, the controller 325 determines a first-illumination-level that represents an illumination level of the first-image, and also determines a second-illumination-level that represents an illumination level of the second-image. For instance, the controller 325 can determine an illumination-level by calculating the average intensity of pixels in an image. Such an average intensity can be the mean or median value for the intensity of pixels in the image, for example.

The controller 325 can then process the first-illumination-level and the second-illumination-level to determine the illumination contribution of the one or more of the LEDs 310-319. For instance, the controller 325 can determine the illumination contribution of one or more of the LEDs based on: the ratio of the first-illumination-level to the second-illumination-level; or the difference between the first-illumination-level and the second-illumination-level. In this way, the difference between the first-illumination-level and the second-illumination-level can represent the illumination contribution of LEDs 310-319 that are on when the first-image is acquired and are off when the second-image is acquired (i.e., those that are in the first set but not in the second set).

In one implementation, the second set of LEDs 310-319 may include more than one LED. That is, a plurality of LEDs can be on when the second-image is acquired. The second set of LEDs 310-319 may include a majority of the LEDs (e.g., more than half of them), and in this example includes all except one of the LEDs 310-319. Including a plurality of LEDs 310-319 in the second set can maintain the absolute value of the determined second-illumination-level at a sufficiently high level such that negative effects due to noise in the determined illumination level and / or the sensitivity of the camera can be mitigated against. That is, if only a single LED were in included in the second set in an alternative implementation, then the determined second-illumination-level could be heavily influenced by noise. This is because the absolute level of the determined second-illumination-level would be lower than if the second set of LEDs included a plurality of LEDs, and therefore any noise / ambient light will have proportionally a greater effect.

As a further way of mitigating against background noise / ambient light in the acquired first-image and second-image, the controller 325 can receive a third-image of the surface 333, which is acquired while the surface 333 is not illuminated by any of the LEDs 310-319. The controller 325 can then process the first-image, the second-image and the third-image to determine the illumination contribution of one or more of the LEDs 310-319. For instance, the controller 325 can determine: a first-illumination-level that represents an illumination level of the first-image (acquired while a first set of LEDs are illuminated, which may be all of the LEDs); determine a second-illumination-level that represents an illumination level of the second-image (acquired while a second set of LEDs are illuminated, which may be a subset of the LEDs); and determine a third-illumination-level that represents an illumination level of the third-image (acquired while none of the LEDs are illuminated). The controller 325 can then subtract the third-illumination-level from the first-illumination-level to determine an effective-first-illumination-level of the first-image; that is, one with the effects of noise / ambient light reduced. Similarly, the controller 325 can also subtract the third-illumination-level from the second-illumination-level to determine an effective-second-illumination-level of the second-image; that is, again, one with the effects of noise / ambient light reduced. The controller 325 can then process the effective-first-illumination-level and the effective-second-illumination-level to determine the illumination contribution of the one or more of the LEDs. For instance, using any of the methods for determining illumination contribution described herein.

It will be appreciated that the controller 325 can receive a plurality of second-images, acquired while the surface 333 is illuminated by second sets of LEDs that represent different subsets of the plurality of LEDs. That is, different LEDs can be turned off when different second-images are acquired such that different LEDs can be isolated. Then, the illumination contributions of different LEDs, in this example all of the LEDs, can be determined. In this way, the controller 325 can process the first-image and the plurality of second-images to determine a plurality of illumination contributions of a plurality of the LEDs 310-319. The controller 325 can then determine the light-source-control-signalling 334 for each of the LEDs 310-319 based on the determined plurality of illumination contributions of the plurality of the LEDs.

With reference to the example that is described above where the second set of LEDs 310-319 includes all except one of the LEDs, a separate second-image can be acquired when each individual LED is turned off in turn. That is, a first-image can be acquired when all of the LEDs are on. A first second-image can be acquired when each of the second to the tenth LEDs are on, and the first LED is off. A second second-image can be acquired when the first and each of the third to the tenth LEDs are on, and the second LED is off. *Et cetera,* up until a tenth second-image is acquired when each of the first to the ninth LEDs are on, and the tenth LED is off. In this way, using the processing described elsewhere in this document, the illumination contribution of each of the ten individual LEDs 310-319 can be determined. It will be understood that similar principles apply if there is a different number of LEDs than 10.

In some examples, the light-source-control-signalling that is disclosed herein is for setting a current level that is to be applied to the one or more LEDs when the eye tracking system is in use. In one implementation, each of the LEDs 310-319 is initially operated (i.e., turned on) by supplying it with a predetermined current such as 10mA. However, as discussed above, providing the predetermined nominal current to each LED will not necessarily mean that each LED 310-319 provides a contribution to the illumination of the surface 333 such that the surface is evenly illuminated. If the controller 325 determines that the illumination contribution of one or more of the LEDs is not at a desired level, then it can set the light-source-control-signalling to adjust the current (by reducing or increasing the current) that is provided to those LEDs when they are on in order to achieve more even overall illumination when all of the LEDS are one when the eye tracking system is subsequently in use.

In one implementation, the controller 325 can determine the light-source-control-signalling for one or more of the LEDs by: comparing: (i) the determined illumination contribution of one or more of the LEDs with (ii) one or more threshold values, to determine an illumination-error-value. The one or more threshold values may include a maximum threshold value and a minimum threshold value that together define a target range of the illumination contribution of the LED. In which case, the determined illumination-error-value can be: a positive value that represents the difference between the determined illumination contribution and the maximum threshold, if the determined illumination contribution is greater than the maximum threshold; a negative value that represents the difference between the determined illumination contribution and the minimum threshold, if the determined illumination contribution is less than the minimum threshold; or zero if the determined illumination contribution is between than the maximum threshold and the minimum threshold.

The one or more threshold values are not necessarily the same for each of the plurality of LEDs - for example, due to the relative positioning of the different LEDs 310-319 they may have different individual targets (as defined by the threshold values) such that when all of the LEDs are on (as they will be when the eye tracking system is in use), together they provide an even illumination of the user's eye.

Once the controller 325 has calculated the illumination-error-value, it can determine the light-source-control-signalling based on the illumination-error-value. For instance, if the controller 325 determines that the determined illumination contribution of an LED is too low, then it can set the light-source-control-signalling such that the LED is provided with a higher current when the eye tracking system is subsequently in use. Similarly, if the controller 325 determines that the determined illumination contribution of an LED is too high, then it can set the light-source-control-signalling such that the LED is provided with a lower current when the eye tracking system is subsequently in use.

In some examples, the controller 325 can compare the determined illumination contribution of the one or more of the LEDs with a maximum-safety-threshold value. Such a threshold represent a maximum illumination level that is considered safe for the user, and can be set according to an eye safety standard. In this way, the light-source-control-signalling can be set such that it does not represent a current that would result in illumination that is too high and therefore unsafe for the user's eye. The maximum threshold value for the determined illumination contribution can be determined by performing analysis for the particular design of the eye tracking system. If the controller 325 would otherwise determine that the light-source-control-signalling should be set such that the illumination level of one or more LEDs would be higher than the maximum-safety-threshold value, then the controller 325 can set the light-source-control-signalling such that the current provided to the LEDs causes the illumination level of the LEDS to be substantially equal to the maximum-safety-threshold value. In this way, the current that is provided to an LED results in illumination that is as close to being even as possible, while still being safe for the user's eye.

As a non-limiting example, the controller 325 can set the light-source-control-signalling based on the illumination-error-value by using a look-up table. Such a look-up table can store a relationship between illumination-error-values and an associated change to the light-source-control-signalling. For instance, an associated change to the light-source-control-signalling may be an increase or decrease by a predetermined amount (i.e., a relative change to the light-source-control-signalling) or may be an absolute value for the light-source-control-signalling that is expected to provide the desired level of illumination. As another non-limiting example, the controller 325 can apply an algorithm to the illumination-error-value in order to determine the light-source-control-signalling. As a further non-limiting example, if the illumination-error-value is a negative value, then the controller 325 can iteratively increase the value of the light-source-control-signalling until the determined illumination contribution of the LED reaches an acceptable value. Similarly, if the illumination-error-value is a positive value, then the controller can iteratively decrease the value of the light-source-control-signalling until the determined illumination contribution of the LED reaches an acceptable value. Such an iterative approach can be based on newly acquired images or can be based on a simulation of how the determined light-source-control-signalling will change the illumination contribution of the affected LEDs, as described in more detail below.

In one example, the determined light-source-control-signalling for each individual LED 310-319 can be stored in memory, for example as part of a configuration file, such that the eye tracking system can subsequently be operated according to the values of the light-source-control-signalling that are stored in the configuration file.

In a yet further implementation, the first set and the second set of LEDs 310-319 may each include only a single LED such that the individual LEDs are turned on sequentially, as different images are acquired, and the controller can readily determine the illumination contribution of each of the LEDs 310-319.

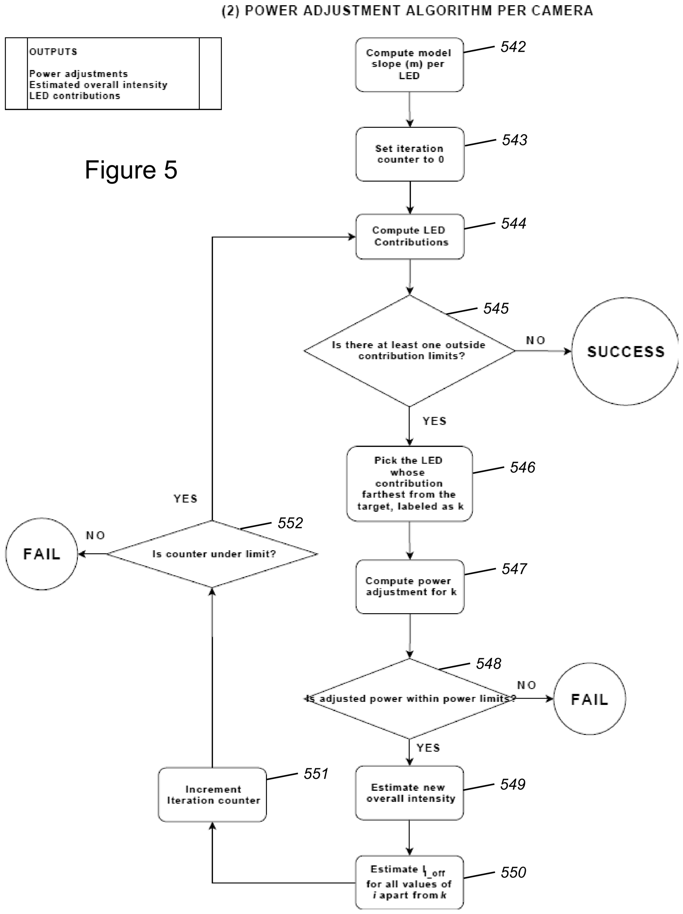

Figure 5 illustrates an example embodiment of an method for calibrating an eye tracking system according to the present disclosure. Since the method is computer implemented, it will be described as an algorithm with respect to Figure 5.

Before the algorithm begins at step 542, one or more of the following initiation procedures are performed:
1. The process reads the following parameters from memory:
   o number of LEDs
   o algorithm parameters
      - nominalLedPower
      - minLedPower
      - maxLedPower
      - minIntensityAllLedsOnWithoutBackground
      - maxNumberSaturatedPixelsPerImage
      - minContributionPerLed
      - maxContributionPerLed
      - targetContributionForAdjustedLeds
      - maxNumberIterations
2. Get
   o serialized data from images; [e.g. a first-image, a plurality of second-images, and a third-image are acquired, as described above]
   o detect features in images; [e.g. an illumination level is determined for each of the acquired images, as described above]
3. Map intensities
   o Intensity when all LEDs are ON (other lens cup off): Iₒₙ; [e.g. register the illumination level of the first-image as Iₒₙ]
   o Intensity when all LEDs are OFF (other lens cup off): I_{off}; [e.g. register the illumination level of the third-image as I_{off}]
   o Intensity when single LED (index i) is off and rest ON (other lens cup OFF): I_{i_off}; [e.g. register the illumination levels of the i second-images as I_{i_off}]
4. Check if all ON LED image (e.g. the first-image described above that is acquired while all of the LEDs are on) meets the following criteria:
   o Iₒₙ - I_{off} > minIntensityAllLedsOnWithoutBackground
   o Number of saturated pixels in each image < maxNumberSaturatedPixelsPerImage
   If it does not, then the process fails and does not go any further.
5. Compute LED contributions
   o Individual contribution of LED i: cᵢ = (Iₒₙ - I_{i_off}) / (Iₒₙ - I_{off})
6. Check if each LED's individual contribution is within bounds [minContributionPerLed, maxContributionPerLed]
   If yes, produce merge file with nominal LED power levels and exit with success. [That is, no adjustment to the illumination levels of the LEDs is required and the light-source-control-signalling can be provided on the basis of the initial control signals for the LEDs, as represented by the nominalLedPower parameters.]
   Otherwise, calculate light-source-control-signalling that will cause power adjustments for the LEDs according to the algorithm flow of Figure 5.

At step 542 of Figure 5, the algorithm involves computing a model slope (m) per LED. In this example, the algorithm assumes that the LED illumination has a linear relationship with LED power. So the slope of this linear function can be calculated by m = I / P, where I is single LED intensity and P is the power of LED. As will be discussed below, the model slope (m) can be used to calculate a required power adjustment at step 547.

At step 543, the algorithm initiates a counter by setting it to zero.

At step 544, the algorithm computes the LED contributions. For the first iteration, when step 544 is performed for the first time, this may simply involve using the individual contributions of the LEDs (cᵢ) that have already been calculated based on the acquired images using the following formula: cᵢ = (Iₒₙ - I_{i_off}) / (Iₒₙ - I_{off}), where Iₒₙ, I_{i_off} and I_{off} are as defined above under step 3 of the initiation procedures. Step 544 will be performed differently for the subsequent iterations, as will be discussed below.

At step 545, the algorithm determines if there is at least one LED contribution that is outside the contribution limits - i.e. is not between minContributionPerLed and maxContributionPerLed (as defined above under step 1 of the initiation procedures). For the first iteration, the algorithm should determine that there is at least one LED contribution that is outside the contribution limits, such that the algorithm will move on to step 546. This is because the same check will have been performed at step 6 of the initiation procedures, as described above. For subsequent iterations however, step 545 may determine that there is not at least one LED contribution that is outside the contribution limits (i.e., all of the LED contributions are considered acceptable), in which case the algorithm can end and be considered as a successful conclusion of the algorithm of Figure 5. As will be appreciated from the description that follows, the output of the algorithm can be the power adjustments, the estimated overall intensity and the LED contributions, at least some of which are an example of light-source-control-signalling that is suitable for setting the intensity of light provided by the LEDS when the eye tracking system is in use.

At step 546, for each LED that has an illumination contribution that is outside the contribution limits, the algorithm calculates the difference between the illumination contribution and the targetContributionForAdjustedLeds parameter. For example, if there are 10 illuminators / LEDs and the illumination should be evenly distributed, then the targetContributionForAdjustedLeds parameter can be set such that the target contribution for each illuminator / LED is 10%. This is an example of how to calculate an illumination-error-value. In another example, the algorithm can calculate the illumination-error-value as the difference between the illumination contribution and the closest one of the contribution limits (i.e., either minContributionPerLed or maxContributionPerLed). The algorithm then identifies the LED that has the largest difference (i.e. is furthest from the contribution limits) and labels that LED as *k.*

At step 547, the algorithm calculates the required power adjustment for LED k. This can be performed by applying the model slope (m) to the illumination-error-value, in order to calculate the required power adjustment that will achieve a required change in illumination contribution of LED k such it is expected to provide an acceptable illumination contribution (i.e. one that is within the contribution limits). The application of such a model slope (m) can be considered as applying an algorithm to the illumination-error-value in order to determine the power adjustment for LED k (which is an example of, or can be part of, light-source-control-signalling).

At step 548, the algorithm then determines whether or not the power adjustment that is calculated at step 547 is within acceptable power limits for the LED k. This can involve comparing the calculated power adjustment with a maximum and a minimum threshold, such as the minLedPower and the maxLedPower parameters that are identified above under part 1 of the initiation procedure. In this context, the adjustment that is required for the LED k, and the corresponding thresholds, can be implemented as power values (as implied here) or current values (as discussed above). For instance, the nominalLedPower may correspond to 10mA, the minLedPower may correspond to 5 mA, and the maxLedPower may correspond to 20 mA. If the calculated power adjustment is not within the acceptable power limits for the LED k, then the algorithm ends and the calibration routine is considered as a fail. If the calculated power adjustment is within the acceptable power limits for the LED k, then the algorithm moves on to step 549.

At step 549, the algorithm simulates the illumination that is provided by all of the LEDs using the power adjustments that are computed at step 547. In this way, the algorithm can estimate the new overall image intensity that would be achieved by all of the LEDs being on, thereby updating the value of Iₒₙ, based on a simulation of the LEDs being operated according to the updated light-source-control-signalling.

At step 550, the algorithm simulates the illumination that is provided when all of the LEDs apart from one are on, for each of the LEDs excluding a simulation for the *k*^{th} LED being on, using any relevant power adjustments that have been computed at step 547 in a previous iteration. In this way, the algorithm can estimate the new image intensity I_{i_off} that would be achieved when a single LED (index i) is off and the rest are on for all values of i apart from k, based on a simulation of the LEDs being operated according to the updated light-source-control-signalling.

At step 551, the algorithm increments the iteration counter, and then at step 552 the algorithm checks that the iteration counter is less than a maximum number of iterations (as implemented by the maxNumberIterations parameter that is identified above under part 1 of the initiation procedure). If the iteration counter is greater than the maximum number, then the algorithm ends and the calibration routine is considered as a fail. If the iteration counter is not greater than the maximum number, then the algorithm returns to step 544.

In a similar way to that described above, at step 544, the algorithm computes the LED contributions, but this time based on a simulation of the LEDs being operated with adjusted power levels. For the second and each subsequent iteration, at step 544 the algorithm calculates the individual contributions of the LEDs using the following formula: cᵢ = (Iₒₙ - I_{i_off}) / (Iₒₙ - I_{off}), using any updated values for Iₒₙ and I_{i_off} that have been calculated at steps 549 and 550. The algorithm then moves on to step 545 and continues in the same way that is described above until the calibration ends as a success or fail.

If the algorithm ends as a success, then as discussed above, the output of the algorithm can include one or more of the power adjustments for each LED that has been identified as the *k*^{th} LED, the estimated overall intensity (the latest value for Iₒₙ) and the LED contributions (cᵢ). At least the power adjustments for each LED that has been identified as the *k*^{th} LED can be considered as light-source-control-signalling, on the basis that they are for setting the intensity of light provided by the respective LEDs sources when the eye tracking system is subsequently used such that the LEDs will more evenly illuminate the user's eye.

In some applications, the calibration algorithm can include the following additional processing step. As part of the initiation procedures or the iterative loop that is shown in Figure 5, the algorithm can determine the minimum and / or the maximum value for the intensity of pixels in the acquired images. If the minimum value is less than a minimum threshold or if the maximum value is greater than a maximum threshold, then the algorithm may end the calibration routine as a fail. Optionally, when the algorithm determines that the calibration routine has failed, for whatever reason, it can provide an output signal (such as an output log file) that is indicative of the reason for the failure.

In some examples, the entire processing of Figure 5 (and the associated initiation procedures) can be performed iteratively to determine updated-light-source-control-signalling. Indeed, any of the processing described herein that determines light-source-control-signalling can be performed iteratively to determine updated-light-source-control-signalling. Such an iterative approach can result in further improvements to the evenness with which a user's eyes can be illuminated when the eye tracking system is in use, albeit at the cost of additional processing time and additional processing resource.

Such an iterative approach can be implemented by:
- driving the first set of LEDs according to the determined light-source-control-signalling and receiving an updated-first-image of the surface, acquired while the surface is illuminated by the first set of the plurality of LEDs;
- driving the second set of LEDs according to the determined light-source-control-signalling and receiving an updated-second-image of the surface, acquired while the surface is illuminated by the second set of the plurality of LEDs;
- processing the updated-first-image and the updated-second-image to determine an updated illumination contribution of one or more of the LEDs; and
- determining updated-light-source-control-signalling for one or more of the LEDs based on the determined updated illumination contribution of the one or more of the LEDs.

It will be appreciated that the plurality of LEDs can be repeatedly driven according to the updated-light-source-control-signalling to iteratively determine updated-light-source-control-signalling until an end condition is satisfied. Such an end condition may be a predetermined number of iterations or when any measured parameter that represents the illumination of the surface satisfies a predetermined condition.

As an optional addition to examples disclosed herein, especially ones that implement an iterative approach, the algorithm can perform a check that the LEDs are providing the expected level of illumination when they are operated using the light-source-control-signalling (or any updated-light-source-control-signalling). For instance, the algorithm can simulate the illumination that is expected to be provided by the LEDs using the light-source-control-signalling, or any updated-light-source-control-signalling, in a similar way to that described with reference to step 547 of Figure 5. The algorithm can then compare the results of the simulation with determined illumination characteristics based on an image that is acquired when the LEDs are operated with the same light-source-control-signalling, or any updated-light-source-control-signalling. In this way, the algorithm can calculate a simulation-error-value based on the comparison, which represents the degree of similarity between the expected and the actual illumination of the LEDs when their operation is adjusted with a view to improving the evenness of the illumination. If the simulation-error-value is greater than a threshold value, then the algorithm can provide an output signal, such as an output log file, that represents the difference. In this way, non-linearities in operation of the LEDs, which may be caused by them over-heating for example, can be identified.

Although the majority of the above description is presented with reference to head mounted eye tracking systems, it will be appreciated that the features described herein can equally be implemented for any type of eye tracking system including remote eye tracking systems. Any eye tracking system that has a plurality of light sources and a camera, even if they are not collocated, can benefit from the calibration routines that are described herein. Furthermore, the calibration routine does not need to be implemented in a test environment. That is, the same processing and algorithms can be implemented while the eye tracking system is in a position of use for tracking a user's eye (i.e. at run-time), and can result in improvements to the evenness of the illumination of the user's eye and therefore an improvement to the eye tracking operation.

As a further example, although the above examples are described with reference to processing an entire image of a user's eye, in some examples there can be advantages to splitting an image of a user's eye into two or more segments (for example, four quadrants), and processing each of those segments separately. This may especially be the case if there is more than one degree of freedom associated with the LEDs, such as if their position or orientation with respect to the user's eye can be adjusted (in addition to being able to adjust the intensity of the light that is provided by the LED as discussed in detail above).

### CLAUSES

Clause 1. An eye tracking system (300) comprising:
a plurality of light sources (310-319) that are arranged to illuminate a user's eye when the eye tracking system is in use; and
a controller (325) configured to:
   receive a first-image of a surface (333), acquired while the surface (333) is illuminated by a first set of the plurality of light sources;
   receive a second-image of the surface, acquired while the surface (333) is illuminated by a second set of the plurality of light sources, wherein the second set of light sources is different to the first set of light sources;
   process the first-image and the second-image to determine an illumination contribution of one or more of the light sources (310-319); and
   determine light-source-control-signalling (334) for one or more of the light sources (310-319) based on the determined illumination contribution of the one or more of the light sources, wherein the light-source-control-signalling (334) is for setting the intensity of light provided by the respective light sources (310-319) when the eye tracking system (300) is in use.

Clause 2. The eye tracking system (300) of clause 1, wherein:
the first set of the plurality of light sources (310-319) comprises all of the light sources;
the second set of the plurality of light sources (310-319) comprises a subset of the light sources;
   and:
the controller (325) is configured to:
   process the first-image and the second-image to determine an illumination contribution of the light sources that are not included in the second set of the plurality of light sources; and
   determine light-source-control-signalling (334) for the light sources that are not included in the second set of the plurality of light sources based on the determined illumination contribution of the light sources that are not included in the second set of the plurality of light sources.

Clause 3. The eye tracking (300) system of any preceding clause, wherein the controller is configured to:
receive a plurality of second-images, acquired while the surface (333) is illuminated by seconds sets of the plurality of light sources that represent different subsets of the plurality of light sources (310-319);
process the first-image and the plurality of second-images to determine a plurality of illumination contributions of a plurality of the light sources; and
determine light-source-control-signalling for the light sources based on the determined plurality of illumination contributions of the plurality of the light sources.

Clause 4. The eye tracking system (300) of any preceding clause, wherein the controller (325) is configured to:
determine a first-illumination-level that represents an illumination level of the first-image;
determine a second-illumination-level that represents an illumination level of the second-image; and
process the first-illumination-level and the second-illumination-level to determine the illumination contribution the one or more of the light sources (310-319).

Clause 5. The eye tracking system (300) of clause 4, wherein the controller (325) is configured to:
determine the first-illumination-level by calculating the average intensity of pixels in the first-image; and
determine the second-illumination-level by calculating the average intensity of pixels in the second-image.

Clause 6. The eye tracking system (300) of clause 5, wherein the controller (325) is configured to:
determine the illumination contribution of one or more of the light sources based on: a ratio of the first-illumination-level to the second-illumination-level; or the difference between the first-illumination-level and the second-illumination-level.

Clause 7. The eye tracking system (300) any preceding clause, wherein the controller (325) is further configured to:
receive a third-image of the surface (333), acquired while the surface (333) is not illuminated by any of the plurality of light sources (310-319); and
process the first-image, the second-image and the third-image to determine the illumination contribution of one or more of the light sources (310-319).

Clause 8. The eye tracking system (300) of clause 7, wherein the controller (325) is configured to:
determine a first-illumination-level that represents an illumination level of the first-image;
determine a second-illumination-level that represents an illumination level of the second-image;
determine a third-illumination-level that represents an illumination level of the third-image;
subtract the third-illumination-level from the first-illumination-level to determine an effective-first-illumination-level;
subtract the third-illumination-level from the second-illumination-level to determine an effective-second-illumination-level; and
process the effective-first-illumination-level and the effective-second-illumination-level to determine the illumination contribution of the one or more of the light sources (310-319).

Clause 9. The eye tracking system (300) of any preceding clause, wherein the light-source-control-signalling is for setting a current level that is to be applied to the one or more light sources when the eye tracking system is in use, and wherein the controller (325) is configured to:
determine the light-source-control-signalling (334) for the one or more of the light sources (310-319) by:
comparing: (i) the determined illumination contribution of one or more of the light sources with (ii) one or more threshold values, to determine an illumination-error-value; and
determining the light-source-control-signalling (334) based on the illumination-error-value.

Clause 10. The eye tracking system (300) of any preceding clause, wherein the controller is configured to:
set the light-source-control-signalling based on the illumination-error-value by:
using a look-up table; or
applying an algorithm to the illumination-error-value.

Clause 11. The eye tracking system (300) of any preceding clause, wherein the controller (325) is configured to:
drive the first set of light sources according to the determined light-source-control-signalling and receive an updated-first-image of the surface (333), acquired while the surface (333) is illuminated by the first set of the plurality of light sources;
drive the second set of light sources according to the determined light-source-control-signalling and receive an updated-second-image of the surface (333), acquired while the surface (333) is illuminated by the second set of the plurality of light sources;
process the updated-first-image and the updated-second-image to determine an updated illumination contribution of one or more of the light sources; and
determine updated-light-source-control-signalling for one or more of the light sources (310-319) based on the determined updated illumination contribution of the one or more of the light sources.

Clause 12. The eye tracking system (300) of clause 11, wherein the controller (325) is configured to repeatedly drive the plurality of light sources (310-319) according to the updated-light-source-control-signalling to iteratively determine updated-light-source-control-signalling until an end condition is satisfied.

Clause 13. A method of operating an eye tracking system (300), the eye tracking system comprising a plurality of light sources (310-319) that are arranged to illuminate a user's eye when the eye tracking system (300) is in use, the method comprising:
receiving a first-image of a surface (333), acquired while the surface (333) is illuminated by a first set of the plurality of light sources;
receiving a second-image of the surface (333), acquired while the surface (333) is illuminated by a second set of the plurality of light sources, wherein the second set of light sources is different to the first set of light sources;
processing the first-image and the second-image to determine an illumination contribution of one or more of the light sources (310-319); and
determining light-source-control-signalling (334) for one or more of the light sources (310-319) based on the determined illumination contribution of the one or more of the light sources, wherein the light-source-control-signalling is for setting the intensity of light provided by the respective light sources when the eye tracking system (300) is in use.

Clause 14. A test rig (435) for an eye tracking system (300), wherein the eye tracking system (300) comprises a plurality of light sources (441) that are arranged to illuminate a user's eye when the eye tracking system (300) is in use, and wherein the test rig (435) comprises:
a first surface (438) of known reflectivity for simulating the reflectivity of the user's eye;
a second surface (439) of known reflectivity, which is less reflective than the reflectivity of the first surface (438), for simulating the reflectivity of regions of the user's face around their eye; and
a mount (440) for the eye tracking system (300) such that, when the eye tracking system (300) is located in the mount (440), the plurality of light sources (441) illuminate at least the first surface (438).

Clause 15. The test rig (435) of clause 14, wherein:
the first surface (438) is generally circular and wherein the first surface is coplanar with the second surface; and the second surface (439) surrounds the generally circular first surface (438), wherein the test rig (435) preferably comprises two first surfaces (438), one for each eye.

## Claims

1. A method for calibrating an illuminance level of a plurality of light sources (441) in an eye tracking system (300), the method comprising the steps of:
setting a first current level to a one or more of the plurality of light sources (310-319),
illuminating at least a first surface (438) by the one or more of the plurality of light sources (310-319),
determining illumination contribution of the one or more of the plurality of light sources (310-319) on the illuminance level of the eye tracking system (300),
configuring the illuminance level of the eye tracking system (300) by setting an adjusted current level on the one or more of the plurality of light sources (310-319).

2. The method of claim 1, wherein setting of the adjusted current level is on the condition that the determined illumination contribution of the one or more of the of the plurality of light sources (310-319) is above a maximum threshold or below a minimum threshold.

3. The method of claims 1-2, wherein setting of the adjusted current level is based on a power adjustment model, wherein the model defines a linear relationship between the illumination and the current level of each and one of the respective one or more of the plurality of light sources (310-319).

4. The method of claims 1-3, wherein the illumination contribution of the one or more of the plurality of light sources (310-319) on the illuminance level of the eye tracking system (300) when setting the first current level is determined by the steps of:
receiving a first image of the surface while the surface (438) is illuminated by a first set of the plurality of light sources (441),
receiving a second image of the surface while the surface (438) is illuminated by a second set of the plurality of light sources (441), wherein the second set of the plurality of light sources (441) if different from the first set of the plurality of light sources (441),
determining a brightness of the first image and a brightness of the second image,
determining the illumination contribution of the first set of light sources (441) on the illuminance level of the eye tracking system (300) by subtracting the brightness of the second image from the brightness of the first image.

5. The method of claim 4, wherein the method further comprises:
determining the brightness of the first image by calculating the average intensity of pixels in the first image; and
determining the brightness of the second image by calculating the average intensity of pixels in the second image.

6. The method of claims 1-5, wherein the surface (438) comprises a surface of a test rig (435), a surface of an eye of a user of the eye tracking system (300) or a larger portion of a face of the user of the eye tracking system (300) that includes at least one of the eyes of the user.

7. The method of claims 1-6, wherein the adjusted current level based on the determined illumination contribution is set not to exceed a maximum safety threshold.

8. The method of claims 4-7, wherein the method further comprises:
receiving a further image of the surface (438), acquired while the surface (438) is not illuminated by any of the one and more plurality of light sources (310-319); and
processing the first image, the second image and the further image to determine the illumination contribution of one or more of the light sources (310-319).

9. The method of claims 1-8, wherein the method, optionally, comprises arranging the eye tracking system (300) to be located in a mount of a test rig (435), wherein the mount is located in a specified location and orientation with respect to a reflective target (457).

10. A test rig (435) for calibrating an eye tracking system (300), wherein the eye tracking system (300) comprises a plurality of light sources (441) that are arranged to illuminate a user's eye when the eye tracking system (300) is in use, and wherein the test rig (435) comprises:
a reflective target (457) comprising a first and a second surface (438, 439), wherein the first surface (438) is of known reflectivity for simulating the reflectivity of the user's eye and the second surface (439) is of known reflectivity, which is less reflective than the reflectivity of the first surface (438), for simulating the reflectivity of regions of the user's face around their eye; and
a mount (440) for the eye tracking system (300) such that, when the eye tracking system (300) is located in the mount (440), the plurality of light sources (441) illuminate at least the first surface (438).

11. The test rig (435) of claim 10, wherein the first surface (438) is generally circular.

12. The test rig (435) of claims 10-11, wherein the first surface is coplanar with the second surface, and/or wherein the second surface (439) surrounds the generally circular first surface (438).

13. The test rig (435) of claims 10-12 wherein the test rig (435) comprises two first surfaces (438), one for each eye.

14. The test rig (435) of claims 10-13 wherein the reflective target (457) is movable relative to the mount (440).

15. A system for calibrating an illuminance level of a plurality of light sources (441) of an eye tracking system (300), the system comprising:
a test rig (435) according to any of the claims 10-14;
an eye tracking system arranged in the test rig (435);
wherein the eye tracking system (300) comprises a controller (325) configured to perform a method of any of claims 1-9.
